# EUROPEAN PATENT APPLICATION

(11) **EP 1 867 382 A2**
(43) Date of publication of application: **19.12.2007**
(21) Application number: 06731453.4
(22) Date of filing: 29.03.2006
(51) Int. Cl.: B01D 53/86

(54) **METHOD FOR DECOMPOSITION AND REMOVAL OF ORGANIC COMPOUND IN AIR USING PLATINUM AS MAIN CATALYSIS, METHOD FOR FORMATION OF PHOTOCATALYTIC ARTICLE AND PHOTOCATALYTIC LAYER, AND PHOTOCATALYST**

(30) Priority: 29.03.2005 JP 2005093356; 30.03.2005 JP 2005097065; 30.03.2005 JP 2005097068
(71) Applicant: Kabushiki Kaisha Zen World, Shizuoka-shi, Shizuoka, 420-8033 (JP); Matsui, Nobuyuki, Shizuoka-shi, Shizuoka, 420-0867 (JP)
(72) Inventor: TAGATA, Genichi, 4311112 (JP)
(74) Representative: Rupp, Christian
(86) International application number: PCT/JP2006/307506
(87) International publication number: WO 2006/112281

(57) **Abstract**

This invention provides various technologies to maximize an air cleaning effect, utilizing photocatalyst materials such as titanium di-oxide.

It is possible for the photo catalyst material of the present invention to have an effect of air cleaning not only in day time but also at night, making use of titanium di-oxide as main photocatalyst and platinum as auxiliary catalyst. The former is a photocatalyst material and the latter works to have an effect of having organic compounds adsorbed and decomposed.

## Description

### BACKGROUND OF THE INVENTION

The present invention relates to various technologies to maximize the effect of cleaning the air using a photocatalyst agent such as titanium dioxide. Specifically it relates to the following technologies.

That is, by using platinum as a main catalyst and titanium dioxide as a auxiliary catalyst, it can propose a new technique for enabling a harmful organic compound in air to be removed not only during day time but also at night.

In addition the present invention especially relates to photocatalyst agents, especially to photocatalyst agents which can be easily applied onto the surface of the base substance such as window panes.

Moreover, the present invention relates to the method of forming the photocatalyst layer exciting the photocatalyst agent and decomposing and removing the dirt and a harmful organic material that adheres on the base substance by the light such as ultraviolet rays.

In addition, the present invention relates to the photocatalyst agent which is installed on base substances such as plastic boards and the glass boards and excites the photocatalyst compound and removes and decomposes dirt that adheres to this base substance and harmful organic material by the light such as ultraviolet rays.

There are a lot of conventional technologies related to the technique for removing a harmful organic material in air by using the photocatalyst material of the titanium dioxide.

Specifically, as for applying the photocatalyst material to the indoor pane, the method of common uses such as dipping, atomization, the spin coating, the roll coatings, and doctor blade method was done conventionally.

The handily membrane-making method of the photocatalyst film by coating and drying by heating of the photocatalyst paints or (without baking it) by coating with a binder is disclosed (For instance, see Japanese Laid-open Patent Application No.H11-323188).

Moreover, as a method of removing dirt and a harmful organic material that adheres to the surface of the base substance, the method of decomposing and removing it by using the photocatalyst compound is conventionally known.

In such cases, in order to decompose the organic material such as dirt, it is necessary to irradiate the light such as ultraviolet rays with this organic material touched the photocatalyst compound, so if the photocatalyst compound is not exposed to the surface, there is no effect.

Therefore, in the prior art, after the surface coating agent that contains the photocatalyst compound and the binder agent or (according to circumstances) a liquid photocatalyst agent that contains an organic solvent is spread directly on the base substance, the photocatalyst compound was exposed to the surface by wiping a binder agent off, and the photocatalyst layer was formed (See Japanese Laid-open Patent Application No.2004-230263)

### SUMMARY OF THE INVENTION

Conventionally, as a main method for air purification, the photocatalyst reaction that uses the titanium dioxide has been considered.

On the fine day, there is a light source of 350~1500 µW/cm2 in the sun light of natural light close to inside pane. However, conventional method has a fault that the purification function cannot work as a means of the photocatalyst in nighttime since it did not work without light.

And it needs some energy such as artificial light, which is the waste of the resource.

In the photocatalyst that uses the titanium dioxide of the prior art, there is a problem of the light source which is needed to have a function to wash off the dirt such as NO x of outside walls of a building and the road.

Moreover, in the prior art, the photocatalyst is contained in the binder, and the photocatalyst is not spread enough on the surface of the binder with it exposed. So there was a problem that the effect of the photocatalyst was not fully produced since the organic material elimination rate is lowered when the amount of the binder was large.

The present invention provides the photocatalyst article that solves the above-mentioned problem.

There was a problem in the prior art that it cannot decompose or remove polluted organic material enough. In the prior art, when you spread the surface agent on the base substance where the photocatalyst layer is formed, and the base substance have ruggednesses bigger than the particle size of the photocatalyst compound or with the hole like the screen door, the photocatalyst compound and the binder agent collect in the concave portion and the hole of the base substance when the binder agent is wiped off since it doesn't expose the photocatalyst compound enough to the surface.

The purpose of the present invention is to solve the above-mentioned problem and to provide the method of forming the photocatalyst layer by exposing the photocatalyst compound enough to the surface and letting it cling to there, even if there is a ruggedness bigger than particle size of the photocatalyst compound in the surface of the base substance or the there is a hole in the base substance.

In addition, when the photocatalyst compound-the diameter is a very small grainy of about 6-10 nanometer- is mixed with a binder agent and an organic solvent and made liquid in order to spread it on the base substance, the particle of photocatalyst compounds coheres mutually and it becomes a big particle (Hereafter, "Secondary particle").Because these secondary particles are localized on the base substance, in previous art, some part on the base substance was not spread by the photocatalyst compound and it could not fully remove or decompose the organic material such as dirt.

The present invention was developed to solve the pre-mentioned problem and it has aimed to provide the photocatalyst agent that can equally disperse the particle of the photocatalyst compound on the base substance by suppressing the formation of the secondary particle.

The present invention was devised by the repeated examination to solve the problems of prior arts. And by changing the viewpoint of the conventional idea of the photocatalyst which uses titanium dioxide, the method of the photocatalyst using platinum as a main catalyst was produced.

In a word, this method can solve the problem by compensating for the previous fault of the photocatalyst and producing function of adsorption and resolution of platinum enough.

Specifically, as follows:
(1) Method of using platinum as a main catalyst, wherein the air cleaning function is provided continuously for a long time not only during day time but also at night. It is based on the technical thought that using the platinum as a main photocatalyst and titanium dioxide as a auxiliary catalyst.
   Specifically by mixing the binder with platinum and the titanium dioxide and coating the mixture on the base substances, the platinum of 0.05-50% to the content of the titanium dioxide adsorbs an organic compound at the normal temperature even when there is no light. Then platinum adsorbs and decomposes an organic compound in air.
   So while the platinum adsorbs and decomposes an organic compound in air, when leaving it as it is, the platinum is covered with the decomposed materials in several hours and then becomes a catalyst poison which doesn't adsorb or decompose an organic compound.
   And in daytime when there is sunshine, the material covered with the platinum surface is oxidized by the photocatalyst and the catalyst poison is removed. In this way, platinum that lost the catalyst poison recovers the ability of adsorption and resolution.
   Therefore, in daytime platinum work effectively together with the titanium dioxide that performs as photocatalyst and in nighttime platinum adsorbs and decomposes an organic compound in air.
(2) Method of using platinum as a main catalyst, characterized in being able to decompose and remove organic compound in air for 24 hours, described in (1) wherein air cleaning function is provided continuously for a long time not only during day time but also at night,
   On the one hand, platinum decomposes and adsorbs an organic compound in air at nighttime without light, and the other hand, the titanium dioxide utilizes the sun light of less than 390 nm of wavelength in daytime and causes the photocatalyst reaction, oxidizing the catalyst poison adhering around the platinum and removing it.
(3) Method of using platinum as a main catalyst described in (1), wherein air cleaning function is provided continuously for a long time not only during day time but also at night, decomposing and removing organic compound in air.
   In order to increase the effect of the photocatalyst supported by platinum (thereafter we call it "the platinum supported super photocatalyst" because the main catalyst is platinum), the platinum and the titanium dioxide should be touched with air and the particle of the platinum and the titanium dioxide should be properly mixed and supported.
   Therefore, in order to coating it easily, the diatom soil from 20 nm to 100 nm of minute powder is added to a binder, wherein vegetable oil that is the binder assistance material is added and if necessary, the ethylene glycol is added as antifreeze used also for the cold latitudes, and also the taurine and the catechin are added as a diffusion material.
(4) Method of using platinum as a main catalyst described in (1), wherein air cleaning function is provided continuously for a long time not only during day time but also at night, decomposing and removing organic compound in air.
   More exactly, by coating and extending the platinum supported super photocatalyst about 5-100 nm on materials by using a spatula made of the resin like the way cleaning the pain glass, and then wiping it off with toweled cloth made of the unwoven cloth, the platinum supported titanium dioxide to can touch with air.
(5) Method of using platinum as a main catalyst described in (1), wherein air cleaning function is provided continuously for a long time not only during day time but also at night, decomposing and removing organic compound in air.
   More exactly, method of coating the platinum supported super photocatalyst on the indoor side of the pane that can store the maximum amount of ultraviolet rays in indoors enough to remove and decompose the indoor organic compounds.
   The necessary coated area for the building, if converted into the floor space, requires more than 1/7 of coated area, and if converted into the capacity, requires more than 0.08 square meters for one cubic meter.
(6) Method of using platinum as a main catalyst described in (1), wherein air cleaning function is provided continuously for a long time not only during day time but also at night, decomposing and removing organic compound in air.
   More exactly, method of coating and extending the platinum supported super photocatalyst about 5-100 nm on materials by using a spatula made of the resin like the way cleaning the pain glass, and then wiping it off with toweled cloth made of the unwoven cloth. Thereafter the glass becomes transparent because the platinum supported super photocatalyst can be coated in 10-30 nm on a base substance of pain.
(7) Method of using platinum as a main catalyst described in (1), wherein air cleaning function is provided continuously for a long time not only during day time but also at night, decomposing and removing organic compound in air.
   More exactly, when coating it on the tile or the concrete block and the outside wall material, etc. used as a base substance for the inside and outside of the building that the transparency is not needed, the first process is to spread the binder as one of two kinds of sprayed liquid and then coat the platinum supported super photocatalyst during the half dried condition.
(8) Method of using platinum as a main catalyst described in(1), wherein air cleaning function is provided continuously for a long time not only during day time but also at night, decomposing and removing organic compound in air.
   When it is used for products by baking on the tile or the concrete block or the outside wall material used for the inside and outside of the building and it manufactures, the efficiency of the platinum supported super photocatalyst doesn't change as long as the temperature is under 400°C.
(9) Method of using platinum as a main catalyst described in (1), wherein air cleaning function is provided continuously for a long time not only during day time but also at night, decomposing and removing organic compound in air.
   More exactly, to promote the resolution of the nitrogenous substance, the ceric oxide that can oxidizing and reducing the nitrogenous substance using the oxidation heat of the titanium dioxide and the oxidation heat of platinum is added to the platinum supported super photocatalyst and promotes the resolution of NOx.
(10) Method of using platinum as a main catalyst described in (1), wherein air cleaning function is provided continuously for a long time not only during day time but also at night, decomposing and removing organic compound in air.
   Because an organic compound in air is adsorbed and decomposed on the platinum side and do redox of oxygen on titanium dioxide side by the platinum supported super photocatalyst, the pollen of the cause of the causative agent in a chic house and hay fever in air is decomposed and the decomposition speed of the excrement of the tick that is the cause of atopic dermatitis, the atopic asthma, and the atopy rhinitis become rapid.
(11) Method of using platinum as a main catalyst described in (1), wherein air cleaning function is provided continuously for a long time not only during day time but also at night, decomposing and removing organic compound in air.
   Because the platinum supported super photocatalyst has a water-repelling quality, the dirt of inorganic is not easy to attached.

Application products of platinum supported super photocatalyst used for method of using platinum as a main catalyst described in claim 1, wherein air clearing function is provided continuously for a long term not only during day time but also at night, decomposing organic compound in air, and making it remove, which is a material for cleaning, the platinum supported super photocatalyst surface coating material which contains and soaks in the platinum supported super photocatalyst surface coating agent, and a platinum supported super photocatalyst products that had the material for wiping off, the material for cleaning of above-mentioned is the one of cleaning the surface of the base substance such as panes, the above-mentioned platinum supported super photocatalyst surface coating material is the one to spread the above-mentioned platinum supported super photocatalyst surface coating agent on surface of the above-mentioned base substance after cleaning the surface of the base substance above-mentioned with the above-mentioned cleaning material.

The material for the above-mentioned wiping off is the one to wipe off the surface of the above-mentioned base substance after the above-mentioned platinum supported super photocatalyst surface coating agent is spread on the surface of the above-mentioned base substance.

Moreover, to achieve the purpose pre-mentioned, the photocatalyst article in claim 12 is a photocatalyst article with material for cleaning, a photocatalyst surface coating material that contains and soaks in photocatalyst surface coating agent, and a material for wiping off.

Moreover, according to the photocatalyst article described in claim 13, in the photocatalyst article in claim 12, a material for cleaning, a photocatalyst surface coating material, and a material for wiping off are sealed up respectively in wrapping bag. And the above mentioned sealed-up wrapping bag which contains photocatalyst surface coating agent is made of the material of an optical impermeability.

Moreover, according to the photocatalyst article described in claim 14, in the photocatalyst article described in claim 12 or claim 13, the base substance is the glass and the porous quality unwoven cloth of super-extra fine chemical fiber is used in a material for cleaning, a photocatalyst surface coating material, and a material for wiping off.

Moreover, the means in claim 15 is a method of coating the supported photocatalyst sealer agent that contains a binder agent and an organic solvent at least on the base substance, and forming the photocatalyst underlayer on the base substance, then coating surface coating agent that at least contains photocatalyst compound and dispersing agent on this the photocatalyst underlayer and consequentially photocatalyst layer is formed on the above-mentioned photocatalyst underlayer.

The means in claim 16, in the invention described in claim 15, is a method of forming photocatalyst layer containing either of modified epoxy resin or oxidation silicon (SiO2).

Moreover, the means in claim 17 is a photocatalyst agent of the composition that contains the dispersing agent, which at least contains photocatalyst compound and the binder agent and auxiliary binder agent that contains oil element.

Moreover, the means in claim 18 is a photocatalyst agent with the photocatalyst compound of 0.1-20 mass %, the binder agent of 0.1-20 mass %, the auxiliary binder agent that contains the oil element of 20-70 mass %, and the dispersing agent of 0.01-1 mass %.

The means in claim 19 is a photocatalyst agent with the photocatalyst compound of 0.1-20 mass %, the binder agent of 0.1-20 mass %, the auxiliary binder agent that contains the oil element of 20-70 mass %, the abrasive agent of 0.1-1 mass %, and the dispersing agent of 0.01-1 mass %.

The means in claim 20 is a photocatalyst agent described in claim 17-19 and the dispersing agent is a photocatalyst agent of the composition that contains the amino acid.

The means in claim 21 is a photocatalyst agent described in claim 17-20 and the dispersing agent is a photocatalyst agent of the composition that contains the saccharose fatty acid ester.

The means in claim 22 is a photocatalyst agent described in claim 17-21 and the dispersing agent is a photocatalyst agent of the composition that contains the polyphenols.

The present invention is based on the technical thought that using the platinum as a main photocatalyst.

Specifically by mixing the binder with platinum and the titanium dioxide and coating the mixture on the base substances, the platinum of 0.05-50% to the content of the titanium dioxide adsorbs an organic compound at the normal temperature even when there is no light. Then platinum adsorbs and decomposes an organic compound in air.

So while the platinum adsorbs and decomposes an organic compound in air, when leaving it as it is, the platinum is covered with the decomposed materials in several hours and then becomes a catalyst poison which doesn't adsorb or decompose an organic compound.

And in daytime when there is sunshine, the material covered with the platinum surface is oxidized by the photocatalyst and the catalyst poison is removed. In this way,
the air cleaning function is efficiently achieved not only during day time but also at night by combining the capacity of platinum which removes the catalyst poison with the ability of photocatalyst.

The light of less than 390 nm of wavelength is enough for titanium dioxide to act photocatalyst reaction, so this effect of the invention always becomes possible for 24 hours by using the usual sun light inserted from outdoor in the room continuously.

Even there's no light, it is possible that a clean effect of air continues for a long time by using platinum as a main catalyst and doing platinum of far exceeding a conventional level to support for the titanium dioxide which is the photocatalyst

The effective clean function is achieved by coating and extending the platinum supported super photocatalyst about 5-100 nm on materials by using a spatula made of the resin like the way cleaning the pain glass, and then wiping it off with toweled cloth made of the unwoven cloth in order to touching the platinum supported titanium dioxide with air.

More exactly, it is confirmed that the effective clean function is fully achieved in the condition that the coated area for the building, if converted into the floor space, is more than 1/7 of coated area, and if converted into the capacity, more than 0.08 square meters for one cubic meter.

In addition, it is confirmed the transparency of the clear glass of the base substance was secured by using platinum supported photocatalyst to be thinness 10-30 nm.

The most effective coating is realized in accordance with the property of the base substance. Specifically, when coating it on the tile or the concrete block and the outside wall material, etc. used as a base substance for the inside and outside of the building that the transparency is not needed, the first process is to spread the binder as one of two kinds of sprayed liquid and then coat the platinum supported super photocatalyst during the half dried condition.

It is found that when it is used for products by baking on the tile or the concrete block or the outside wall material used for the inside and outside of the building and it manufactures, the efficiency of the platinum supported super photocatalyst doesn't change as long as the temperature is under 400 °C. Therefore simple construction to such a base substance is realized.

And the possibility of the air cleaning to various materials was shown, by finding out the method of promoting the resolution of the nitrogenous substance : the ceric oxide that can oxidizing and reducing the nitrogenous substance using the oxidation heat of the titanium dioxide and the oxidation heat of platinum is added to the platinum supported super photocatalyst and promotes the resolution of NOx.

This is an effective invention to a lot of symptoms because the pollen of the cause of the causative agent in a chic house and hay fever in air is decomposed and the decomposition speed of the excrement of the tick that is the cause of atopic dermatitis, the atopic asthma, and the atopy rhinitis become rapid. More exactly, an organic compound in air is adsorbed and decomposed on the platinum side and do redox of oxygen on titanium dioxide side by the platinum supported super photocatalyst.

It is found that since the platinum supported super photocatalyst has a water-repelling quality, the dirt of inorganic is not easy to be attached. So it is difficult to decrease the efficiency due to the dirt of the surface

According to photocatalyst material described in claim 12, it is a material for cleaning, the photocatalyst surface coating material which contains and soaks in the photocatalyst coating agent, and a platinum supported super photocatalyst products that had the material for wiping off.
The cleaning material for above-mentioned is the one of cleaning the surface of the base substance such as panes.
The above-mentioned photocatalyst surface coating material is the one to spread the above-mentioned platinum supported super photocatalyst surface coating agent on surface of the above-mentioned base substance after cleaning the surface of the base substance above-mentioned with the above-mentioned cleaning material.

The material for the above-mentioned wiping off is the one to wipe off the surface of the above-mentioned base substance after the above-mentioned platinum supported super photocatalyst surface coating agent is spread on the surface of the above-mentioned base substance. In short, by a simple procedure : the cleaning material → photocatalyst surface coating material → the material for the wiping off, it is exposed to the surface of the base substance of the indoor panes, where the photocatalyst compound is bonded and then wiped off by wiping material. So the air in the room can be cleaned by the photocatalyst effect of decomposing the floating organic material.

Moreover, according to the photocatalyst article described in claim 13, in addition to photocatalyst article in claim 12, a material for cleaning, a photocatalyst surface coating materia, and a material for wiping off are sealed up respectively in wrapping bag. So without using the coating apparatus such as spraying for the surface of the base substance of the indoor panes, it is possible to adhere the photocatalyst compound easily by the simple step of the cleaning material → photocatalyst surface coating material → the material for the wiping off. Moreover, because the sealed-up wrapping bag which contains photocatalyst surface coating agent is made of the material of an optical impermeability, the deterioration of photocatalyst surface coating material which contains and soaks in the photocatalyst surface coating agent can be prevented.

Moreover, according to the photocatalyst article described in claim 14, in addition to the effect of invention described in claim 12 or claim 13, because the porous quality unwoven cloth of super-extra fine chemical fiber is used in the materials for cleaning, a photocatalyst surface coating, a photocatalyst surface coating materia, and a material for wiping off, it can prevent from damaging the surface of the glass that is the base substance.

Moreover, in the prior art, the photocatalyst is contained in the binder, and the photocatalyst is not spread enough on the surface of the binder with it exposed. So there was a problem that the effect of the photocatalyst was not fully produced since the organic material elimination rate is lowered when the amount of the binder was large.

Moreover, the method of forming the photocatalyst layer by the present invention forms the photocatalyst underlayer on the base substance, and the photocatalyst layer was formed on this photocatalyst underlayer. So even if there is a big ruggedness in the surface of the base substance compared with the particle size of the photocatalyst compound or there is a hole in the base substance, the photocatalyst compound is exposed to the surface and it can cling and then the photocatalyst efficiency works.

In addition, in the present invention, the photocatalyst agent, the binder agent, and the oil element are at least contained. And since a liquid photocatalyst agent that has the auxiliary binder agent contains dispersing agent if necessary, the particle of the photocatalyst compound is not mutually coherent, so it is prevented that the photocatalyst compound is localized on the base substance. Then it is possible to equally disperse the particle of the photocatalyst compound on the base substance by suppressing the formation of the secondary particle.

Moreover, the photocatalyst agent contains the amino acid, the saccharose fatty acid ester, and polyphenols as a dispersing agent, it is possible to improve the decentralization of the particle of the photocatalyst compound that contains the photocatalyst material.

### Brief description of the drawings

Fig. 1 shows the change in the formaldehyde concentration by measuring more than 24 hours every 30 minutes in laboratory.
Fig. 2 is an outline and squint chart that shows the composition of the photocatalyst article of the present invention.
Fig. 3 is an outline cross section by 2-2 lines of Fig. 2.
Fig. 4 is an outline cross section by 3-3 lines of Fig. 2.
Fig. 5 is an outline cross section by 4-4 lines of Fig. 2.
Fig. 6 is an outline and squint chart that shows the use of sealing up wrapping bag that involves the cleaning material of Fig. 2.
Fig. 7 is an explanation chart to describe the inside of Fig. 6.
Fig. 8 shows the method of forming the photocatalyst layer of the present invention. (a) is a cross section that shows the part after the photocatalyst underlayer agent is spread, (b) is a cross section that shows the part after the surface coating agent is spread, and (c) is a cross section that shows the part after dried.
Fig. 9 is the cross section that shows expanding part spread a photocatalyst agent according to one embodiment of the present invention on the base substance. (a) shows a state immediately after coating. (b) shows a state wiping by wiping material.
Fig. 10 is the cross section that shows expanding part that is spread the photocatalyst agent of other embodiments of the present invention on the base substance. (a) shows the state immediately after coating. (b) shows the state that is wiped off by wiping material.

In these drawings, A is a photocatalyst article, and 4 is a cleaning material. 5 is a he photocatalyst surface coating material, and 6 is a material for wiping off. 1 is a base substance, and 2 is the photocatalyst underlayer agent. 20 is the photocatalyst underlayer, and 21 is a binder agent. 3 is a surface coating agent, and 30 is a photocatalyst layer. 31 denotes a photocatalyst compound, and 32 denotes a dispersing agent. A' denotes photocatalyst composite layer, 1a denotes a base substance, 3a denotes a coating layer, 4a denotes a photocatalyst layer, 11a denotes a photocatalyst compound, 12a denotes a dispersing agent, 14a denotes a binder agent, 15a denotes a auxiliary binder agent, and 16a denotes a abrasive agent.

### DETAILED DESCRIPTION OF THE INVENTION

Hereinafter is described a specific method of decomposing and removing organic compounds in air in the present invention by using platinum as a main catalyst.

First, the present invention started with the investigation of why decomposion and removal of organic compounds in air is not easily achieved though it can be achieved theoretically when various research and development had been performed. The prior art is on the basis of a conception of decomposing and removing an organic compounds in air through oxidation and reduction reactions that occur when the air is cleaned by using the photocatalyst material of the titanium dioxide.

In a word, there was a situation not to achieve decomposion and removal of organic compounds in air like the theory yet though the development was advanced concerning the method of forming the photocatalyst layer, the material that can be effectively applied on the base substance, and the technique for equally distributing photocatalyst material particles by the dispersing agent.

The inventor paid attention to the following point in the process of investigation : He focused on the fact that when the platinum of a minimum amount is added to the photocatalyst material as an auxiliary agent, a clean effect of air continues repeatedly for a certain length of time even without light at night. In a word, if a clean effect of air is achieved only by the photocatalyst, the effect cannot be produced in the state without light. So there must be a cause for the continuous clean effect except for the photocatalyst reaction.

The inventor finally found out that a small amount of platinum added as an auxiliary agent also works as oxidation-reduction catalyst.

However, there was a problem that the cleaning effect did not continue effectively if the catalyst poison (material that obstructs the reaction on the catalyst) is formed on this surface and not remove continuously, because the surface is covered with the resolution material when platinum works as a catalyst.

To overcome this difficulty, by the repeated examinations, the continuous air cleaning function became possible for a long time without light by continuously removing the catalyst poison on the platinum surface by the application of oxidation and reduction reactions of photocatalyst materials which decompose and remove organic compounds.

Thus, the inventor uses platinum as the main catalyst that works to clean the air, and embodied the new idea to use the photocatalyst material of the titanium dioxide as supplement material to remove the catalyst poison on the platinum surface, and eventually completed the present invention.

To be specifice, using platinum as a main catalyst means adding proper amount of platinum to the photocatalyst material of titanium dioxide so that it can function so efficiently as to decompose and remove organic compounds and clean air. As for platinum, in the prior art, it was added only a small amount even if added. But in the present invention, the amount of added platinum is assumed to be 0.05-50% to the content of the titanium dioxide, more preferably, 5-30%. In a word, it means the platinum plays a main role in carrying out the air cleaning function rather than the platinum occupy as main content.

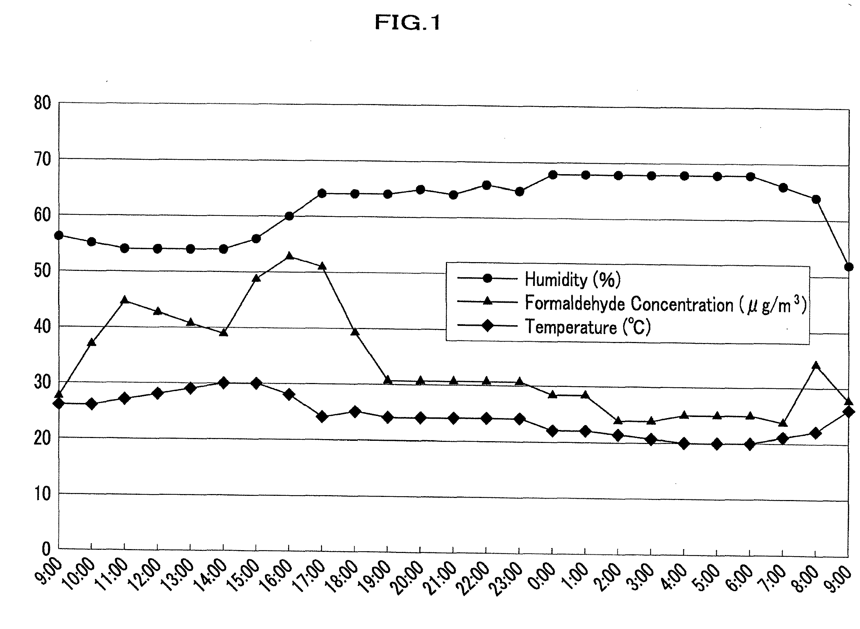

Fig. 1 shows one example of the result of an experiment of how air-purifying function works in the present invention by measuring the indoor formaldehyde concentration every 30 minutes for more than 24 hours. In this experiment, we applied platinum supported photocatalyst of the present invention on a window pane and a wall in the laboratory of the same setting of new furniture in a new building. This figure shows that the formaldehyde concentration remains low not only during day time but also at night. (Though the graph shows the result only until 24 hours elapse, the same result of continuing similar effect is confirmed even if the experiment continues afterwards.)

The graph shows the amount of ultraviolet rays in the room that related to the working of the photocatalyst and the condition of temperature and humidity that relates to generation of formaldehyde, which is shown in relation to measuring time. Specifically, the formaldehyde concentration in the laboratory increases transpiring when the temperature in the room rises and it generates a lot in the daytime of cloudy or rainy weather of the daytime since the amount of ultraviolet rays in the sun light from the outside is small and then photocatalyst doesn't function well. On the other hand, photocatalyst materials such as titanium dioxide work better during daytime when a lot of sun light streams in with large amount of ultraviolet rays.

Having the above in the mind, you can understand from the result of experiment shown in Fig. 1 that rising of the formaldehyde concentration in the laboratory in the morning is attributed to the rise of temperature in the laboratory. Moreover, it is clear that the formaldehyde concentration remains below the certain level, which is due to the air purification function with the photocatalyst material during daytime when the amount of ultraviolet rays in the laboratory is large (Though there are other influences such as the temperature). From evening to night, the formaldehyde concentration increase because the sun light is lost and ultraviolet rays don't enter indoors and therefore the photocatalyst doesn't work.

If interpreting it from above-mentioned assumption, you will predict that the formaldehyde concentration is sure to rise clearly at nighttime (19 o'clock to predawn) when the amount of ultraviolet rays in the laboratory decreases remarkably. However, as the graph of the experiment result shows, the formaldehyde concentration in the laboratory remains low even there is a small amount of ultraviolet rays. (Here, there are other influences such as room temperature, too)

Such a result can be considered to come from the fact that the proper amount of platinum working effeciently to purify the air in the laboratory is supported by the photocatalyst material of the titanium dioxide. Specifically, because of the air purification function by using platinum a catalyst doesn't limited by the amount of ultraviolet rays of indoor, it is considered that it functions effectively at nighttime, too.

In addition, theoretically, the surface of the platinum is covered with the decomposed materials of organic compounds in air, and loses purification function by the catalyst poison. However, unlike a theoretical prediction, the function continues for days. This is presumably because the decomposed materials (catalyst poison) on the appropriately supported platinum are removed from the platinum surface through the photocatalyst reaction.

In a word, the titanium dioxide functioning as a photocatalyst on which platinum is appropriately supported plays a role to remove the catalyst poison that adheres onto the platinum surface through a photocatalytice reaction with ultraviolet rays. To be specific, though the platinum surface on which air is purified at night is covered with the catalyst poison by the adsorption reaction, the platinum surface is cleaned throygh the photocatalyst reaction during daytime of the following day. As a result, the platinum surface can recover the function to purify the air during the next night

Therefore, according to the result shown in Fig. 1, you can effectively use the platinum supported photocatalyst of the present invention. The air cleaning function continues with the photocatalyst material of the titanium dioxide on which platinum as a main catalyst is supported, not only in the case when ultraviolet rays of the sun light exists abundantly but also when photocatalyst reaction doesn't work such as nighttime. Moreover, since the catalyst poison with which the platinum surface is covered at night is properly removed through the photocatalyst reaction of daytime, we can describe that the air cleaning function keeps on working continuously for a long term.

Moreover, as for various pollens that become the causative agents of the hay fever allergy such as the cryptomeria pollen, we cannot expect an enough effect of the decomposing and removing in a conventional photocatalyst material. However, if the platinum is supported as a main catalyst to function air cleaner (for instance, about 10%), and it is confirmed that various pollens are completely decomposed and removed in only several hours.

Hereinafter, the photocatalyst material of the present invention is described according to the drawing.

The base substance (no figure) on which photocatalyst article A is applied is glass (for instance, window pane), or plastic, etc. If there are photocatalyst compounds adhering on the surface of the base substance and ultraviolet rays is irradiated from the back of the base substance (for instance, when the sun ray goes into it through the pane), a plastic material through which the ultraviolet rays penetrates. If ultraviolet rays are irradiated from the surface of the base substance, the plastic material of the ultraviolet ray penetration is not necessary.

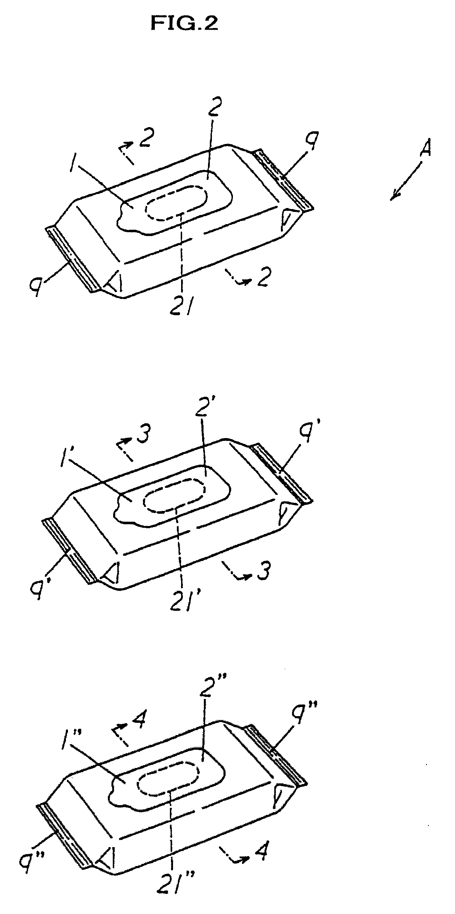

A photocatalyst article A is composed of a cleaning material 4 of the porous unwoven cloth in which the dirt removal agent to clean the surface of the base substance impregnated, the photocatalyst surface coating material 5 (for example, porous unwoven cloth) in which the photocatalyst surface coating agent (compound of abrasive agent, catalyst composition, binder agent, and auxiliary binder agent) is impregnated, and the wiping material 6 for wiping off the surface of the above-mentioned base substance after coating the above-mentioned photocatalyst surface coating agent on the surface of the above-mentioned base substance (wiping material 6, for instance, wipes off the auxiliary binder agent). As shown in Fig.2, the photocatalyst article A is sealed up in wrapping bags 2, 2', and 2" that independently has adhesion opening and shutting parts 1, 1', and 1" respectively. The photocatalyst article A is stored in the wrapping box not shown in the figure.

Sealed up wrapping bag 2 which contain the cleaning material 4 is sealed up by crimp processing section 9 of both ends. Sealed up wrapping bag 2' which contain the photocatalyst compound material 5 is sealed up by crimp processing section 9' of both ends. Sealed up wrapping bag 2" which contain the wiping material 6 is sealed up by crimp processing section 9" of both ends.

And, as described later, by the procedure from cleaning material 4 through photocatalyst surface coating material 5 to wiping material 6, the photocatalyst compounds are exposed and the photocatalyst compound adheres on the surface such as the indoor window panes of the base substance (no figure). As a finish means, it is wiped off so that there is no wipe leavings of the auxiliary binder agent. Eventually the floating organic material is decomposed and the air in the room is cleaned.

In the cleaning material 4, to clean the surface of the base substance (no figure) a dirty removal agent is infiltrated in the porous quality unwoven cloth (for instance, liquid cleaning agent that contains ethyl alcohol, surface-active agent, Tocoferorl, ion exchange water, L Ascolpin acid, and citric acid, etc. as the element).

Before coating the photocatalyst surface coating agent, the dirt of the surface of the base substance such as the indoor window panes (coating side) is removed and especially oil on the surface of the glass is removed with cleaning material 4 made of the porous quality unwoven cloth for cleaning the surface of the base substance , which is impreganated with the dirt removal agent.

A photocatalyst surface coating material 5 is the porous quality unwoven cloth that is impregnated with a mixture of an abrasive agent, photocatalyst compounds, a binder agent, and an auxiliary binder agent.

The cleaning material 4 or the photocatalyst surface coating material 5 can be made by dipping a porous quality unwoven cloth in the dirt removal agent or the photocatalyst surface coating agent.

A wiping material 6 of the porous quality unwoven cloth for wiping off the auxiliary binder agent is used as it is not impregnated.

As a finishing step, an auxiliary binder agent is wiped off for there to be nothing left on the surface of the base substance with a wet cloth or a towel (preferably, it is the one which dampened with the hot water of about 40°C, or whose 1/3 part in the length is put up by water, and the wet part is wrapped by the dry part of 2/3 of remained length, and this dry part become both outer side) that have some moisture (no figure).

Concerning the cleaning material 4, the photocatalyst surface coating material 5, and the wiping material 6, since all of them are made of a super-extra fine chemical fiber, the base substances such as glass surface can be prevented from being damaged.

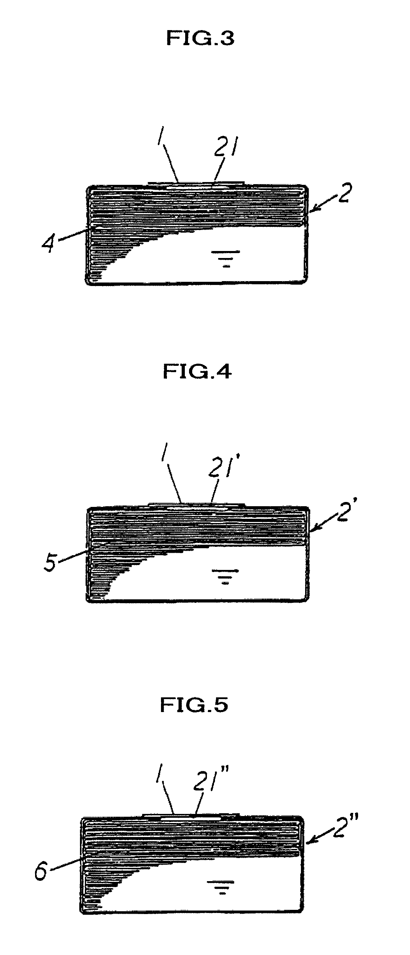

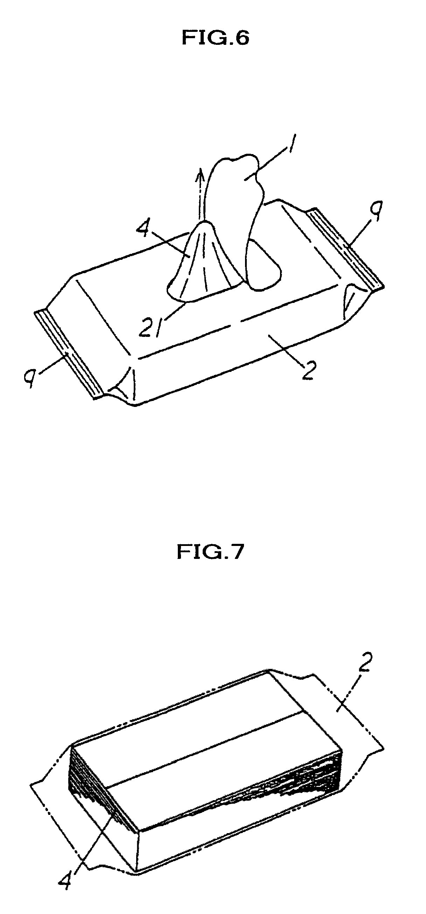

In Fig. 2 and Fig. 3, a sealing up bag 2 is shown that contains plural cleaning materials 4. The cleaning materials 4 of the porous quality unwoven cloth are folded with wimples and wrapped in a sealing up bag 2. A part 1 is the adhesion opening-shutting part where a potion 21 is opened and shut. Around the part 21 that is the back side of opening-shutting part 1 or the corresponding part, flaked-again adhesive is applied, which gives sealed-up lid by opening and shutting repeatedly. By sealing up, cleaning material 4 in sealing up wrapping bag 2 can be prevented from being polluted with mold and miscellaneous germs (see Fig. 2, Fig. 2, Fig. 5, and Fig. 6).

In Fig. 1 and Fig. 3, a sealing up bag 2 ' is shown that contains plural sheets of photocatalyst surface coating materials 5. The photocatalyst surface coating materials 5 of the porous quality unwoven cloth are folded with wimples and wrapped in a sealing up bag 2'. A part 1' is the adhesion opening-shutting part where a potion 21' is opened and shut. Around the potion 21' that is the back side of opening-shutting part 1 or the corresponding part, flaked-again adhesive is spread, which gives sealed-up lid by opening and shutting repeatedly. By sealing up, photocatalyst compound 5 in sealing up wrapping bag 2' can be prevented from being polluted with mold and miscellaneous germs. Especially, sealing up bag 2' that contains photocatalyst compounds is formed of a material of an optical impermeability (for instance, aluminum deposition seat).

The photocatalyst compounds of the photocatalyst surface coating agent tha impregnates photocatalyst surface coating material 5 are as follows:
Titania, titanium dioxide, sol-gel type titanium compound, or metallic oxide such as zinc oxide, oxidation tin, iron oxide, copper oxide, silver oxide, oxidation tungsten, zirconium dioxide, oxidation bismuth, oxidation indium, oxidation cadmium, germanium oxide, nickel oxide, cobalt oxide, oxidation chrome, manganese oxide, vanadium oxide, oxidation niobium, oxidation antimony, strontium titanate,
Especially preferably the titanium dioxide, anatase type titanium dioxide crystal, rutile type titanium dioxide crystal, or those compound or sol-gel type titanium compounds. And as the photocatalyst with photocatalyst surface coating material 5, you can use other materials that show a photocatalytic effect for a visible light.

Not limited to the above-mentioned method, according to the photocatalyst compound of the photocatalyst surface coating agent that impregnates photocatalyst surface coating material 5, at least one kind of the metal or the metallic compound can be fixed to the surface of the photocatalyst particle physically or chemically (See JP, 2000-96800, A.). Among metal or the metallic compound, platinum is the most preferable than gold, silver, copper, iron, cobalt, nickel, chrome, and zinc, etc. By supporting metal on the surface of the photocatalyst, the charge separation of the photocatalyst is promoted and the photocatalyst effect increases. Therefore, when the metal is supported on the surface of the photocatalyst or the photocatalyst is supported on the surface of the metal, both are the photocatalyst compound. The amount of the immobilized metals is preferably 1wt%-50wt% to a whole photocatalyst compound amount.

Moreover, 0.1 wt%s-30 wt% of the photocatalyst is put in an organic solvent such as ethanol. Then stir it enough to make it sol-gel state and add the metal which makes photocatalyst effect larger and mix it enough again. Or you can make it sol-gel state by mixing the metal that makes the photocatalyst effect larger, adding an organic solvent such as ethanol and mixing it enough.

Moreover, though the particle size of the titanium dioxide is not especially limited, it only has to be a particle size of 6nm-10nm of the extent that dissolves easily to an organic solvent. It is desirable to assume the density of the photocatalyst compound contained in the solution of an organic solvent within photocatalyst compound to be 0.1wt%-80wt% . When the densities of the photocatalyst compound are less than 0.1wt%, the effect of the photocatalyst or the power of decomposing contaminant becomes weak, which is undesirable. Moreover, the ultra-fine particle solution of the photocatalyst compound is a liquid acid mixture including the titanium dioxide. The mixture is adjusted to mix the 30-60% of ethyl alcohol, isopropyl alcohol, and an organic solvent such as butyl alcohol, less than 1% of solutions including sulfuric acid and the nitric acid, and 1-30% of anatase or the rutin type titanium alkoxide crystal.

Also, the photocatalyst compounds of the photocatalyst surface coating agent that impregnates photocatalyst surface coating material 5 are formed to a minute powder. The particle size is 6nm-10nm, and is 0.1wt%-30wt% for a whole of photocatalyst surface coating agent amount. If the particle size is larger than 10 nm, it is difficult to merge in the solution of the binder agent (describes later) and the inconvenience is caused that the surface of the base substance after spread becomes cloudy and opaque.

The photocatalyst surface coating agent is composed of the photocatalyst compound, the binder agent, the auxiliary binder agent, and the abrasive agent.

Moreover, if photocatalyst compound is less than 0.1wt% at the pre-mentioned mixing ratio, poisonous substance (organic compound) is not oxidized nor reduced and the photocatalyst reaction cannot be effective. If it is more than 30 wt%, the transparency on the surface of the base substance cannot be provided, which causes inconvenience.

We can make the photocatalyst compound not easy to be oxidized, and avoid quality degradation of the oil element added to the auxiliary binder agent (mentioned later) by adjusting it's pH (unit that displays acidity or alkalinity) to 1-6 pH, adding the surface-activating agent of the common use and maintaining acidity or acidulous. In this adjustment, if pH is less than 1, the acid is so strong that the oily element (recorded later) changes in quality. And when it is six or more, the quality of an oily element cannot be maintained.

The pre-mentioned binder agent acts as a binder that supports the photocatalyst compound. In the binder, the composition or natural paste-for example silicon resin, acrylic fiber resin, fluorine resin, epoxy resin-are used properly. With the dilution liquids such as water and ethyl alcohol, it is formed in liquid solution of a prescribed density that has enough fluidity.

The mixing ratio is adjusted to 0.1 wt%-20 wt% to the whole amount of Photocatalyst surface coating agent, for instance.

When diluting it, the blending quantity is preferably, for example ; the dilution liquid : the binder liquid = 70wt%-99.9wt%:wt%30-0.1wt%.

The pre-mentioned auxiliary binder agent is used to coat photocatalyst compound as thinly as possible on surface of base substance (coating object).

This auxiliary binder agent has oil ingredient, and this oil ingredient is composed of the wax of the natural origin, mineral-spirit solvent, the vegetable oil, the surface-active agent of common use etc. As wax of the natural origin among the oil ingredients, you may choose at least one kind from the following group of materials; calbana-wax, candy lira-wax, comenuca-wax, ouricuri-wax, sugarcane -wax, hohoba-wax, abracate seed-wax, moc-wax, japan-wax, and propolis. These oil ingredients have the water-shedding quality, give gloss to the spread surface and have the characteristic in which plastic is compatible.

Moreover, as an element of the auxiliary binder agent, a mineral spirit solvent is used with a little toxicity. It gives excellent paint film properties, caustic resistance, weather resistance, and quick drying properties, and excellent coating work by the usual coating method of spraying, brushing, and rolling, etc.

The pre-mentioned mineral spirit in the auxiliary binder agent, you may choose at least one kind from the group of materials, mineral turpentine, a white spirit, mineral thinner, and a petroleum spirit, etc.

In addition, the vegetable oil in the pre-mentioned auxiliary binder agent has non-drying oil property, and it is desirable not to easily to oxidizes or change the quality. Specifically, the arbitrary one can be selected as long as it is a material that can be coated thinly and broadly. So you may choose at least one kind from the group of materials, camellia oil, olive oil, sesame oil, soybean oil, safflower oil, colza oil or evening primrose oil, etc.

Moreover, if necessary, we add Ttilhidorokishitolen, Tocoferorl, and Fitin sannad etc. to this photocatalyst surface coating agent as antioxidant materials to prevent the discoloration which originates in the oxidation of pre-mentioned vegetable oil and wax. Other antioxidant materials are ; Phenol material, aromatic amine, Fenotiagen, Getiohosfat, Getiocalbamat, Slfid, and sulfuration Orefin.

Among the vegetable oils, effective for the oxidation prevention are ; L-ascorbic acid fatty acid ester, L-ascorbic acid palmitin acid ester and/or L-ascorbic acid stearic acid ester etc.

Moreover, as an antioxidant of the food additive, you may use ; L-ascorbic acid, L-sodium ascorbate (vitamin C), Ethylenediaminetetraacetic acid Ca2Na, Four ethylene Geamin acetic acids 2Na, Elsolbin acid, Elsolbin acid sodium, Gayac resin, and isopropyl citrate, Nolgehidorogayareticc acid, propyl gallate, and Gebtilhidorokishitolen (BHT), D1-α - Tocoferorl (vitamin E), Btilhiderokishianisorl (BHA), rosemary extraction material, Etokishikin, etc.

As preservative of the food additive; benzoic acid, sodium benzoate, sorbic acid, and potassium sorbate, Debidoro sodium acetate, Paraokishi benzoic acid isobutyl, Paraokishi benzoic acid Isopropil, Paraokishin benzoic acid ethyl, Paraokishin benzoic acid Btil, Paraokishin benzoic acid propyl, propionic acid, sodium propionate, and propionic acid calcium. You may at least add the one kind from among these antioxidants.

Next, we describe the coating method and the photocatalyst surface coating agent on the surface of the base substance of the photocatalyst surface coating agent in the present invention.

The method of exposing the photocatalyst compounds on the surface of the outside layer of the photocatalyst layer includes ;
(1) A liquid photocatalyst surface coating agent is obtained by mixing ultrafine particle solution of photocatalyst compounds, the binder agent, auxiliary binder agent that contains oil ingredients, and the abrasive agent.
(2) The photocatalyst layer is formed on the surface of the base substance by coating on the surface of the base substance and spreading it thinly with the photocatalyst surface coating material 5 that is impregnated with this photocatalyst surface coating agent.
(3) The layer of this photocatalyst compounds, the binder agent, and the auxiliary binder agent is removed with wiping material 6 in order for there not to be no auxiliary binder agent left. As a result, the photocatalyst compounds are exposed on the most outer surface of the photocatalyst layer.

Moreover, the pre-mentioned abrasive agent is formed to be 0.1micron-0.5micron particles with a material of silica or ceramic, and added to the photocatalyst surface coating agent to be mixed.

When particles of the abrasive agent are samller than 0.1 micron, there occurs an inconvenience that both photocatalyst compounds and the binder agents are wiped off and removed when wiping the photocatalyst layer. When it is larger than 0.5 micron, there occurs another inconvenience that the entire photocatalyst layer is removed with this abrasive agent.

In the mixture of this abrasive agent, you may mix it after mixing it with the binder agent and the auxiliary binder agent and the mixing ratio is 10wt%-50wt% in this case.

If an amount of addition of the abrasive agent is less than 10wt%s, there occurs an inconvenience in the coating process because the solidification of the photocatalyst surface coating agent proceeds before the base substance is fully coated. If it is more than 50 wt%, it is difficult to coat it by the photocatalyst surface coating material 5 because it is too water soluble.

The photocatalyst compounds, the binder agent, the auxiliary binder agent, and the abrasive agent are added into a proper container at one time and diluted to a prescribed density with a dilution liquid such as ethyl alcohol or water, then made a solution. Each mixing ratio can be arbitrarily selected in the range of the mixing ratio shown in the present embodiment.

As one example of the photocatalyst surface coating agent, the one listed below is indicated. (Naturally no limitation to this compounding ratio rate.)
- Photocatalyst composition 5wt%
- Binder agent 5wt%
- Binder supplement 65 wt%
- Grinding agent 25 wt%

Add the above materials and mix them evenly, then you can gain a liquid photocatalyst surface coating agent.

The photocatalyst article A is taken out of the wrapping box (Or, wrapping bag) (shown in Fig. 1). You can takes out materials respectively; (1) Take out cleaning material 4 from the potion 21 by peeling off sealed up opening-shutting part 1. (2) Take out photocatalyst surface coating material 5 from the potion 21' by peeling off sealed up opening-shutting part 1'. (3) Take out wiping material 6 from the potion 21" by peeling off sealed up opening-shutting part 1".

The surface of the base substance of the indoor window pane is cleaned by cleaning material 4 that is impregnated with the dirt removal agent. The photocatalyst compounds are exposed and bonded onto the surface of the base substance of the indoor window pane by the photocatalyst surface coating material 5 that are impregnated with the abrasive agent, the catalyst compounds, the binder agent and the compounds of the auxiliary binder agent. An amount of binder agent which unnecessary for exposing and bonding the photocatalyst compounds is removed by wiping material 6, and the photocatalyst compounds are bonded onto the surface of the base substance such as the indoor window panes. Through a series of procedures, the photocatalyst compounds are spread transparently on the surface of the indoor window pane. The state of the spread photocatalyst compounds can be confirmed by only touching the indoor pane lightly by the tip of a finger and feeling roughly.

Though the above-mentioned photocatalyst article doesn't necessarily relate to the platinum supported photocatalyst, it is surely useful when actually using the platinum supported photocatalyst.

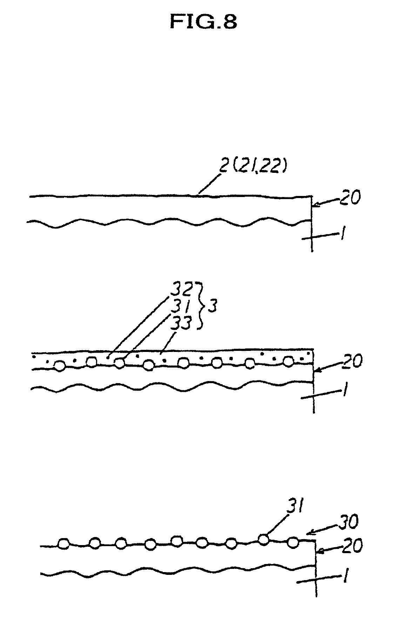

We will describe the embodiment of the method of forming the photocatalyst layer of the present invention in Fig. 8. In the figure, 1 indicates base substance, 2 indicates photocatalyst underlayer agent spread on 1, and 3 indicates the surface coating agent spread on the base substance.

The base substance 1 is an object on which organic materials such as dirt are decomposed and removed and becomes a catalyst support on which photocatalyst compound 31(mention later) are fixed. This base substance 1 includes the unwoven cloth, the screen door, the roll curtain, and the filter of air conditioner, the resin boards, the outer wall material of buildings, tents, tents, and concrete blocks, etc. The photocatalyst underlayer agent 2 includes a binder agent 21 for bonding photocatalyst compound 31, which is mixed with binder agent 21 and organic solvent 22 in a prescribed ratio.

For example, as binder agent 21, we use the silicon oxide (SiO2) of 0.1 mass %-10 mass % mixed with organic solvent 22 such as ethanol and liquidize it.

Alternatively, as binder agent 21, we use the modified epoxy resin of 0.1 mass %-10 mass % mixed with organic solvent 22 that contains a butyl acetate, a cyclohexane, propylene glycol methylic ether, propylene glycol methylic ether acetate and liquidize it. You can mix a dispersing agent 32(mention later) to disperse binder agent 21 as photocatalyst underlayer agent 2.

The surface coating agent 3 includes photocatalyst compound 31 that decomposes the organic material such as dirt. We use mixing of photocatalyst compound 31 of 0.1 mass % - 20 mass % and dispersing agent 32 of 0.01 mass %-1 mass % and liquidize it.

The above-mentioned photocatalyst compound 3 is formed of particles such as titania, titanium dioxide or sol-gel type titanium compound or metallic oxidation compound of zinc oxide, oxidation tin, iron oxide, copper oxide, silver oxide, oxidation tungsten, zirconium dioxide, oxidation bismuth, oxidation indium, oxidation cadmium, germanium oxide, nickel oxide, cobalt oxide, oxidation chrome, manganese oxide, vanadium oxide, oxidation niobium, oxidation antimony, and strontium titanate.

But preferably, the titanium dioxide, and Anatase type Titanium dioxide crystal and Ltil type Titanium dioxide crystal and those compounds or the sol-gel type titanium compound is used.

Moreover, though the particle size of this photocatalyst compound 31 is not especially limited, it has to be a particle size as small as 6nm-10nm so that this photocatalyst compound 31 dissolves easily into an organic solvent. When the particle size is smaller than 6nm, the processing become difficult and cost is high. And when the particle size is larger than 10nm, it is not easy to merge in organic solvent 33 and the surface of base substance 1 become cloudy and the transparence decreases.

The above-mentioned dispersing agent 32 disperses the particle such as photocatalyst compounds 31, for instance, Teanin, the lysine, the glutamic acid, the aspartic acid, and polyphenols such as the saccharose fatty acid esters such as amino acids such as an arginine and professional phosphorus, the saccharose Steffen acid esters, the saccharose stearic acid esters, and the saccharose palmitin acid esters and catechins are used. You may use a natural thing such as extracted material obtained by extracting tea leaf that contains catechins of polyphenols and Teanin of amino acid in element with hot water.

Moreover, you may mix gold and the metals such as silver, copper, platinum, iron, cobalt, the nickel, chrome, and zinc or the metallic compounds with surface coating agent 3 (no figure) . By mixing this metal or the metallic compound, the charge separation with photocatalyst compound 31 is promoted and the degradation characteristic of the organic material by the photocatalyst improves.

In addition, you may mix the ceric oxide with surface coating agent 3 (no figure). The ceric oxide decomposes and removes the organic material as dirt by heat. And by using reactive heat (generation of heat) from the resolution of the organic material generated by decomposing and removing of photocatalyst compound 31, the effect of the resolution removal improves.

Next, we describe the method of forming photocatalyst layer 30. First of all, the above-mentioned photocatalyst underlayer agent 2 is coated on the base substance 1 by using a well-known techniques such as the spray coating method as shown in Fig. 8 (a), then photocatalyst underlayer 20 is formed. This photocatalyst underlayer 20 is so thick that it can adhere to the photocatalyst compound 31, the metal or the metallic compound. And it is formed as thick as about ten nanometer after dried, for example.

Shortly before organic solvent 22 evaporates gradually and photocatalyst underlayer agent 2 is as dry as possible in the half-dried condition after coating the photocatalyst underlayer agent 2, the above-mentioned surface coating agent 3 is coated by using a well-known technique like the spray coating method on the photocatalyst underlayer 20(See Fig. 8 (b)).

The above-mentioned drying method may be either room-temperature drying of leaving the photocatalyst underlayer agent 2 at room temperature or forced drying by using warm air.

The reason why the surface coating agent 3 is coated on the half dried photocatalyst underlayer agent 2 is that the photocatalyst compound 31 is can be bonded on the photocatalyst layer with the binder agent 21. After organic solvent 22 in the photocatalyst underlayer agent 2 or the organic solvent 33 in the surface coating agent 3 is evaporated and dried, the photocatalyst compound 31 can be exposed to the surface as shown in Fig. 8 (c).

Moreover, the purpose for using the modified epoxy resin as binder agent 21 of photocatalyst underlayer agent 2 is that the particle of photocatalyst compounds 31 is bonded steady even if the ruggedness on the surface of base substance 1 is comparatively small. And the purpose of using silicon oxide (SiO2) as binder agent 21 is to restrict the particles of photocatalyst compounds 31 sinking and being buried in the photocatalyst underlayer 20 before organic solvent 22 is dried. You may select the kind of photocatalyst underlayer agent 2 arbitrarily according to the state of interface of base substance 1.

In addition, the purpose of mixing dispersing agent 32 with surface coating agent 3 is to prevent the particle of photocatalyst compounds 31 from agglomerating with one another. Before coating, it need not be stirred shortly before coating since the particle doesn't sink. After coating, the particle adheres to the photocatalyst underlayer 20 uniformly and the photocatalyst layer 30 can be formed since the localization by the agglomeration of the particle is prevented.

Therefore, since surface coating agent 3 doesn't contain binder agent 21 that conventionally bonds the photocatalyst compound 31, the photocatalyst compound 31 can be exposed to the surface even if it isn't wiped off after surface coating agent 3 is spread if the organic solvent 33 is dried. By being mixed with the dispersing agent, it can be bonded onto the photocatalyst underlayer 20 equally. And even if a base substance is not flat and has large ruggedness or the hole is open such as screen door on the surface, the photocatalyst layer 30 can be installed by exposing photocatalyst compound 31 to the most outer surface of the base substance. Therefore, when the organic materials of dirt adhere to the surface, they are decomposed and removed by the excitation of photocatalyst compound 31, if it touches photocatalyst compound 31 and the light such as ultraviolet rays is irradiated under such a condition.

Though the method of forming the photocatalyst layer here doesn't necessarily relate to the platinum supported photocatalyst, it can be naturally applied when the platinum supported photocatalyst is actually used.

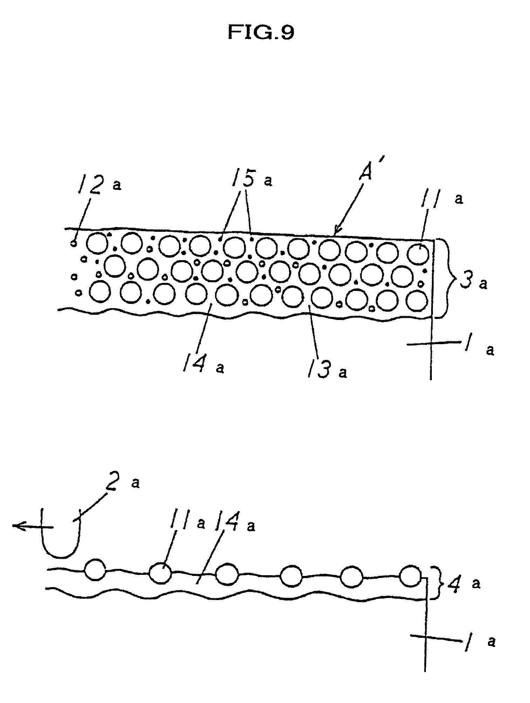

Moreover, the execution of the photocatalyst agent of the present invention is described in accordance with Fig. 9-Fig. 10.

In Fig. 9 (A), A' denotes a photocatalyst composite layer, which is applied on base substance 1 such as walls in window panes like buildings and cars, plastic boards or the solar parts and rooms. Through the photocatalyst reaction on the included photocatalyst compound 11a with the irradiation of the light such as ultraviolet rays, the dirt of a harmful organic material that adheres to base substance 1a is removed after decomposed.

As the photocatalyst composite layer A', photocatalyst compound 11a, dispersing agent 12a, binder agent 14a, and auxiliary binder agent 15a that contains the oil element are mixed with organic solvent 13a such as ethanols at a prescribed rate and it is adjusted to liquid.

The above-mentioned photocatalyst compound 11a is a particulate material. For example, titania, titanium dioxide or sol-gel type titanium compound. Alternatively, metallic oxides such as the zinc oxide, oxidation tin, the silver oxide, the oxidation tungsten, the zirconium dioxide, the oxidation bismuth, the oxidation indium, the oxidation cadmium, the germanium oxide, the nickel oxide, the cobalt oxide, the oxidation chrome, the manganese oxide, the vanadium oxide, the oxidation niobium, the oxidation antimonies, and the strontium titanates. But preferably, the titanium dioxide, and anatase type titania dioxide crystal, rutile type titania dioxide crystal or those compound or sol-gel type titanium compounds are used.

Though the particle size of this photocatalyst compound 11a is not especially limited, though the particle size of this photocatalyst compound 31 is not especially limited, it only has to be a particle size of 6nm-10nm of the extent that dissolves easily to an organic solvent13a. When the particle size is less than 6nm, the processing become difficult and cost is high. And when the particle size is more than 10nm, it is not easy to merge in organic solvent 13a and the surface of base substance 1a become cloudy and the transparence decreases.

The mixing ratio of the photocatalyst compound 11a is preferably as 0.1 mass %-20 mass % for a whole amount of photocatalyst composite layer A'. When it is less than 0.1 mass %, the organic materials such as dirt are not decomposed and the photocatalyst compound 11a is not to be able to work enough as photocatalyst reaction. When it is more than 20 mass%, the inconvenience of not obtaining the transparency on the surface of base substance 1a causes.

The above-mentioned dispersing agent 12a disperses the particles such as made of photocatalyst compounds 11a, for instance, Teanin, the lysine, the glutamic acid, the aspartic acid, and polyphenols such as the saccharose fatty acid esters such as amino acids such as an arginine and professional phosphorus, the saccharose Steffen acid esters, the saccharose stearic acid esters, and the saccharose palmitin acid esters and catechins are used. You may use a natural substance such as extracted material obtained by extracting tea leaf that contains catechins of polyphenols and Teanin of amino acid in element with hot water. The compounding ratio of the photocatalyst compound 12a is preferably as 0.01 mass %-1 mass % for a whole amount of photocatalyst composite layer A'. When it is less than 0.01 mass %, the effect of decentralization is not gained. When it is more than 1mass %, fluidity decreases and coating becomes difficult.

The above-mentioned binder agent 14a bonds photocatalyst compound 11a on base substance 1a. For example, the composition of Silicon system resin, acrylic fiber system resin, fluorine system resin, epoxy system resin, a natural paste are used, and mixed with the photocatalyst agent to become a prescribed density. The mixing ratio of the binder agent 14a is preferably as 0.1 mass %-20 mass % for a whole amount of the photocatalyst composite layer A'. If it is less than 0. 1 mass %, the particles of the photocatalyst compound 11a cannot be adhere onto the base substance enough. When it is more than 20mass %, fluidity decreases and coating becomes difficult.

The above-mentioned auxiliary binder agent 15a is used for coating photocatalyst compound 11a as thinly as possible on surface of base substance 1a (coating object). This auxiliary binder agent 15 contains oil, and this oil element is composed of wax of the natural origin, mineral-spirit solvent, vegetable oil, surface-active agent of common use etc. As wax of the natural origin among the oil elements, you may choose at least one kind from the following group of materials ; calbana-wax, candy lira-wax, comenuca-wax, ouricuri-wax, sugarcane -wax, hohoba-wax, abracate seed-wax, moc-wax, japan-wax, and propolis.

These oil elements have a hydrophobic property, give gloss to the spread surface. And the mixing ratio is preferably as 20 mass %-70 mass % for a whole amount. When it is less than 20mass %, the mass as big as the rice grain that contains the particle of photocatalyst compound 11a can be made. When it is more than 70mass %, an inconvenience is caused that the whole photocatalyst composite layer A' hardens and cannot be spread.

Moreover, you may mix such metal or metallic compounds as composed of gold, silver, copper, platinum, iron, cobalt, nickel, chrome, or zinc with the photocatalyst composite layer A' (no figure). By mixing this metal or the metallic compound, the charge separation with photocatalyst compound 11a is promoted and the degradation characteristic of the organic material by the photocatalyst improves.

In addition, you may mix the ceric oxide with photocatalyst composite layer A' (no figure). The ceric oxide decomposes and removes the organic material as dirt by heat. And by using reactive heat (generation of heat) from the resolution of the organic material generated by decomposing and removing of photocatalyst compound 11a, the effect of the resolution removal improves.

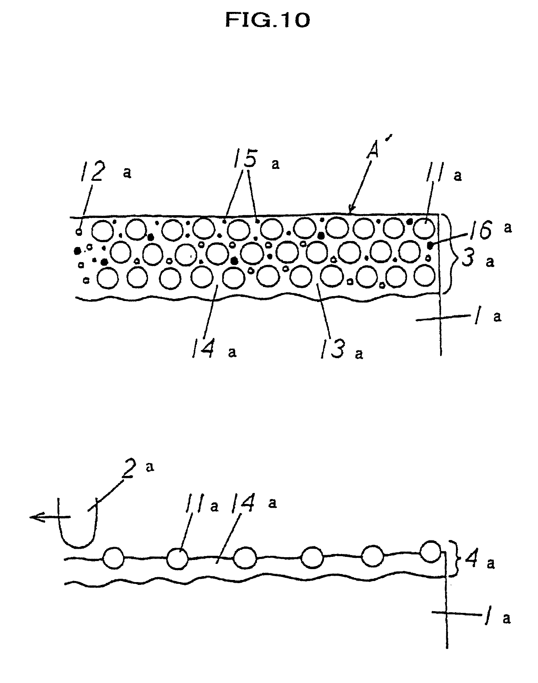

In addition, as shown in Fig. 10 (a), you may mix the abrasive agent 16a with the photocatalyst composite layer A'. After the photocatalyst composite layer A' is coated on base substance 1a as shown in Fig. 10 (b), this abrasive agent 16a plays a role to scratch off and remove the unnecessary extra binder agent 14a, while the photocatalyst composite layer A' is being wiped with the wiping means 2a so that photocatalyst compound 11a is exposed on the surface.

This abrasive agent 16a is made of silica and a material ceramic with its particle sizes formed to be the particulate of 30 nanometer-500 nanometer and are mixed with photocatalyst composite layer A'. If the particle sizes of abrasive agent 16a are less than 30 nanometer, an inconvenience is caused that both the photocatalyst compounds 11a and the binder agent 14a are wiped off and removed when coating layer 3a is wiped off. If the particle sizes of abrasive agent 16a are more than 500 nanometer, an inconvenience is caused that the ratio of photocatalyst compound 11a exposed to the surface decreases, because photocatalyst composite layer A' remains thick even after wiped off.

The mixing ratio rate of abrasive agent 16a is preferably 0.1 mass %-50 Shitiomompaca % to a whole amount of photocatalyst composite layer A'. If it is less than 0.1 mass %, the oil element remains and it cannot be wiped off enough. When it is more than 50 mass %, an inconvenience is caused that amounts of the photocatalyst compound 11a and the binder agent 14a that remains on base substance 1a are not sufficient after wiped off.

Next, we will describe the formation method of the photocatalyst layer 4a on base substance 1a from the photocatalyst composite layer A'. The dirt of the surface on this target base substance 1a for coating photocatalyst composite layer A'(coating side), especially the oil that adheres to the surface, has to be removed sufficiently in advance by applying the dirt removal agent(For instance, Liquid cleaning agent that includes Ethyl alcohol, surface-active agent, Tocofenorl, ion exchange water, L ascorbic acid, and citric acid), or by waste.

As shown in Fig. 9 (a) and Fig. 10 (b), the appropriately mixed photocatalyst agent A is uniformly coated by a well-known technique such as spatula on the whole base substance 1a, and a coating layer 3a is formed on the surface of base substance 1a.

Next, as shown in Fig. 9 (b) and Fig. 10 (b), wiping with a wiping means 2a such as the cloth of a bulky fiber is performed on the coating layer 3a of photocatalyst composite layer A', which is spread on the surface of this base substance 1a and not completely dried. As a result, particles of accumulated photocatalyst compound 11a, binder agent 14a, and auxiliary binder agent 15a is removed from the surface of the coating layer 3a in this photocatalyst composite layer A'. Finally, Photocatalyst layer 4a is formed with the photocatalyst compound 11a exposed on its surface.

In the present invention, we mix the dispersing agent 12a with the photocatalyst composite layer A' in order to disperse uniformly the particles of the photocatalyst compounds 11a in the coating layer 3a on base substance 1a while the photocalayst agent A is being coated. As a result, even after he coating layer 3a is wiped off by the wiping means 2, the effect of decomposing and removing organic materials such as dirt can remain, because the particles of the photocatalyst compounds 11a in the coating layer 3a are uniformly dispersed without agglomeration on the base substance 1a over the whole of base substance 1a on which the photocatalyst layer 4a is formed.

### (Example)

One example of the present invention is described as follows.

First, by using photocatalyst composite layer A' composed of the materials which were mixed with the mixing ratio described in (Eexample of present invention), a photocatalyst layer 4a was formed after wiping with the cloth of a bulky fiber on the coated photocatalyst composite layer A' on the glass board (base substance 1a) cleaned of the surface beforehand.

Moreover, for the purpose of comparison, another photocatalyst agent (comparison example) was formed that did not contain the dispersing agent 12a used fro the present invention, and the photocatalyst layer 4a was formed by a similar method.

### (Example of present invention)

• Photocatalyst composition 11a titania (TiO2), 5 mass %
• Binder agent 14a silicone system resin, 5mass %
• Binder supplement 15a camellia oil solution, 50 mass %
• Dispersing agent 12a Teanin, 0.1 mass %
• Organic solvent 13a ethanol, the remainder

### (Comparison example)

• Photocatalyst composition 11a titania (TiO2), 5mass %
• Binder agent 14a silicone system resin, 5 mass %
• Binder supplement 15a camellia oil solution, 50 mass %
• Organic solvent 13a ethanol, the remainder

Next, by examining the adhering state of the photocatalyst compound 11a on the glass board (base substance 1a) after wiping, It turned out that in (Comparison example), the particles of the photocatalyst compound 11a are not distributed uniformly and that localized particles on the glass board form a secondary particle in which several thousand tens of particle layers are accumulated. On the other hand, in (Eexample of present invention), the formation of the secondary particle is not seen and the particles of the individual photocatalyst compound 11a were dispersed and bonded uniformly on the whole surface of the glass board.

The operation-effect in photocatalyst composite layer A' of the present invention is not limited to this (embodiment) material and mixing ratio. And a similar operation-effect was obtained even if it is made of other materials, and the mixing ratio explained above.

The photocatalyst composite layer A' here is not limited to relate to the platinum supported photocatalyst, and it functions effectively in case of the platinum supported photocatalyst.

## Claims

1. Method of using platinum as a main catalyst, wherein the air cleaning function is produced continuously for a long time regardless of day or night, based on the technical thought that using the platinum as a main photocatalyst and titanium dioxide as a supplementary catalyst, specifically by mixing the binder with platinum and the titanium dioxide and spreading it on the base substances, the amount of the platinum assumed to be 0.05-50% to the content of the titanium dioxide adsorbing an organic compound at the normal temperature even when there is no light platinum adsorbs and resolves an organic compound in air and while the platinum adsorbs and resolves an organic compound in air, when leaving it as it is, the platinum is covered with the material resolved in several hours, then becomes a catalyst poison which doesn't adsorb or resolve it so when there is sunshine, the material covered with the platinum surface is oxidized by the photocatalyst and the catalyst poison is removed, Then, platinum that lost the catalyst poison recovers the capacity of adsorption and resolution so in daytime platinum work effectively together with the titanium dioxide that reacts the photocatalyst, and in nighttime platinum adsorbs and resolves an organic compound in air.

2. Method of using platinum as a main catalyst, **characterized in** being able to 24 hour resolve and remove organic compound in air, described in claim 1, wherein air clearing function is provided continuously for a long term regardless of day or night, that platinum resolves and adsorbes an organic compound in air at nighttime without light, and the titanium dioxide utilize the light of the wavelength of 390 nm or less of the sun light in daytime, oxidizing the catalyst poison attaches to the platinum surroundings causing the photocatalyst reaction and removing it.

3. Method of using platinum as a main catalyst described in claim 1, wherein air clearing function is provided continuously for a long term regardless of day or night, resolving organic compound in air, and making it remove, that platinum and the titanium dioxide coming in contact with air so that the platinum supported super photocatalyst (thereafter it is called the platinum supported photocatalyst) may increase the effect since it is a photocatalyst assumed that platinum is a subject, and additionally, since the platinum particle and the particle of the titanium dioxide should be moderately mixed and supported, as a binder to extend it well when spreading it the diatom soil is made into a minute powder 20 nm to 100 nm, wherein vegetable oil that is the binder assistance material is added and the ethylene glycol is added as antifreeze to use it also for the cold district if necessary, and in adding the taurine and the catechin as a diffusion material.

4. Method of using platinum as a main catalyst described in claim 1, wherein air cleanring function is provided continuously for a long term regardless of day or night, resolving organic compound in air, and making it remove, painting a completed platinum supported super photocatalyst on it with a spatula made of the resin about 5-100 nm as clean the glass and extending it, then being able to have platinum supported titania dioxide to touch with air by wiped off with toweled cloth made of the unwoven cloth.

5. Method of using platinum as a main catalyst described in claim 1, wherein air clearing function is provided continuously for a long term regardless of day or night, resolving organic compound in air, and making it remove, spreading it on the indoor side of the pane that can secure most amounts of ultraviolet rays in indoors enough to remove the indoor organic compound resolving. needing a coated area 1/7 or more which is a necessary coated area for the building if you converts it into the floor space, and needing 0.08 square meters or more for one cubic meter if you convert it into capacity.

6. Method of using platinum as a main catalyst described in claim 1, wherein air clearing function is provided continuously for a long term regardless of day or night, resolving organic compound in air, and making it remove, in which the spread glass becomes transparent and painted with a spatula made of the resin about 5-100 nm as clean the glass and extending it, then being able to have platinum supported titania dioxide to touch with air by wiped off with toweled cloth made of the unwoven cloth.

7. Method of using platinum as a main catalyst described in claim 1, wherein air clearing function is provided continuously for a long term regardless of day or night, resolving organic compound in air, and making it remove, when spreading it on the tile or the concrete block material and the outside wall material, etc. used as a base substance for the inside and outside of the building that the transparency is not needed, firstly spreading the binder, and then putting platinum supported photocatalyst during half dried condition as two kinds of sprayed liquid.

8. Method of using platinum as a main catalyst described in claim 1, wherein air clearing function is provided continuously for a long term regardless of day or night, resolving organic compound in air, and making it remove, and when baked on the tile or the concrete block material and the outside wall material, etc. used for the inside and outside of the building and it manufactures products, if the temperature is 400°C or less, the performance doesn't change even if the platinum supported super photocatalyst is baked.

9. Method of using platinum as a main catalyst described in claim 1, wherein air cleanring function is provided continuously for a long term regardless of day or night, resolving organic compound in air, and making it remove, adding the ceric oxide that can oxidizing reduce the nitrogenous substance by using the oxidation heat of the titanium dioxide and the oxidation heat of platinum to the platinum supported super photocatalyst and promoting the resolution of NOx in order to promote the resolution of the nitrogenous substance.

10. Method of using platinum as a main catalyst described in claim 1, wherein air clearing function is provided continuously for a long term regardless of day or night, resolving organic compound in air, and making it remove, adsorbing and resolveing an organic compound in air by the platinum supported super photocatalyst on the platinum side, resolving the pollen of the cause of the causative agent in a chic house and hay fever in air to do redox of oxygen on titanium dioxide side, and it makes the decomposition speed of the excrement of the tick that is the cause of atopic dermatitis, the atopic asthma, and the atopy rhinitis rapid.

11. Method of using platinum as a main catalyst described in claim 1, wherein air clearing function is provided continuously for a long term regardless of day or night, resolving organic compound in air, and making it remove, **characterized** as not attaching the dirt of inorganic since the platinum supported super photocatalyst is a water-shedding quality.

12. An application product of platinum supported super photocatalyst used for method of using platinum as a main catalyst described in claim 1, wherein air clearing function is provided continuously for a long term regardless of day or night, resolving organic compound in air, and making it remove, which is a material for cleaning, the platinum supported super photocatalyst surface spreading material which contain and soak the platinum supported super photocatalyst surface spreading medicine, and a platinum supported super photocatalyst products that had the material for wiping of,
the material for the above-mentioned cleaning is the one cleaning the surface of the base substance such as panes,
the above-mentioned platinum supported super photocatalyst surface spreading material is the one to spread the above-mentioned platinum supported super photocatalyst surface spreading medicine on surface of the above-mentioned base substance after cleaning the surface of the base substance above-mentioned with the above-mentioned cleaning material,
The material for the above-mentioned wiping off is the one to finish up a wipe of the surface of the above-mentioned base substance aftert the above-mentioned platinum supported super photocatalyst surface spreading medicine is spread on the surface of the above-mentioned base substance.

13. An application products of platinum supported super photocatalyst described in Claim 12,
wherein material for cleaning, material for platinum supported super photocatalyst surface spreading, the material for wiping off are involved to sealing up wrapping bag respectively, and sealing up wrapping bag that involves the above-mentioned platinum supported super photocatalyst surface spreading material is the formation with the material of an optical impermeability.

14. An application products of platinum supported super photocatalyst article described in one of claim 12 and 13, wherein base substance is the glass, the material for cleaning, platinum supported super photocatalyst surface spreading material, and the material for wiping off are all **characterized in** using the porous quality unwoven cloth of a super-extra fine chemical fiber.

15. Method of forming platinum supported super photocatalyst layer described in claim 1 used for using platinum as a main catalyst, further comprising,
decomposing organic compound in air, and making it remove,
spreading the platinum supported super photocatalyst sealer medicine that contains a binder medicine and an organic solvent at least on the base substance and the platinum supported super photocatalyst groundwork layer is formed on the above-mentioned base substance,
spreaing surface spreading medicine that at least contains platinum supported super photocatalyst composition and dispersing agent on to this platinum supported super photocatalyst groundwork layerand platinum supported super photocatalyst layer is formed on the above-mentioned platinum supported super photocatalyst groundwork layer.

16. Method of forming platinum supported super photocatalyst layer described in claim 15, **characterised in** containing either of modified epoxy resin or oxidation silicon (SiO2).

17. The medium of platinum supported super photocatalyst to use platinum as a main catalyst described in claim 1 used for method of resolving organic compound in air, and making it remove,
in platinum supported super photocatalyst medium that contains at least with the binder supplement that contains the platinum supported super photocatalyst composition, the binder medicine, and the oil element and becomes it, it characterizes in the composition it that contains the dispersing agent.

18. The medium of platinum supported super photocatalyst to use platinum as a main catalyst described in claim 1 used for method of resolving organic compound in air, and making it remove that contains

19. The medium of platinum supported super photocatalyst to use platinum as a main catalyst described in claim 1 used for method of resolving organic compound in air, and making it remove that contain
With the platinum supported super photocatalyst composition of 0.1-20 mass %
With the binder medicine of 0.1-20 mass %
With the binder promoting materials that contains the oil element of 20-70 mass %
With the dispersing agent of 0.01-1 mass % and.

20. Dispersing agent is a platinum supported super photocatalyst body described in claims 17-19 wherein the amino acid is contained.

21. Dispersing agent is a platinum supported super photocatalyst body described in claims 17-20 wherein the saccharose fatty acid ester is contained.

22. Dispersing agent is a platinum supported super photocatalyst body described in claims 17-21 wherein polyphenols are contained.
